# EUROPEAN PATENT APPLICATION

(11) **EP 1 658 842 A1**
(43) Date of publication of application: **24.05.2006**
(21) Application number: 04736985.5
(22) Date of filing: 17.06.2004
(51) Int. Cl.: A61K 9/51

(54) **NANOPARTICLES OF POLYOXYETHYLENATED DERIVATIVES**

(30) Priority: 04.07.2003 ES 200301570
(71) Applicant: Advanced in Vitro Cell Technologies, S.L., 08028 Barcelona (ES)
(72) Inventor: ALONSO FERNANDEZ, Maria, José, E-15782 Santiago de Compostela (ES); SÁNCHEZ BARREIRO, Alejandro, E-15782 Santiago de Compostela (ES); CSABA, Noémi, E-15782 Santiago de Compostela (ES)
(74) Representative: ABG Patentes, S.L.
(86) International application number: PCT/ES2004/000282
(87) International publication number: WO 2005/002550

(57) **Abstract**

The invention relates to nanoparticles of polyoxyethylenated derivatives, having a size of less than 1 micrometer, for Lhe administration of pharmaceutically-or cosmetically-active ingredients. The inventive nanoparticles comprise biodegradable polymer, a polyoxycthylene-derived block copolymer and at least one pharmaceutically- or cosmetically-active ingredient. The invention further relates to the method of obtaining the aforementioned nanoparticles and to compositions containing same.

## Description

### FIELD OF THE INVENTION

The present invention relates to nanoparticles (size less than 1 µm) with a new composition, which are suitable for the administration of active molecules. The new composition comprises two polymers: a biodegradable polymer and a polyoxyethylene-derived block copolymer.

### BACKGROUND OF THE INVENTION

Special attention is being given to polymeric nanoparticles due to their interest in improving stability and promoting the transport and controlled release of drugs to certain regions of the organism. The biodegradable polymers most used for their formation are polylactic acid (PLA) derivatives and their copolymers with glycolic acid (PLGA) due to their biodegradability, biocompatibility and innocuousness (Johansen et al., Eur. J. Pharm. Biopharm., 2000, 50, 129-146). Other biodegradable polymers, which also offer a promising future in this line, are polyesters such as poly(ε-caprolactone) (Losa et al., Pharm. Res., 1993, 10,1, 80-87) and polyanhydrides (Mathiowitz et al., Nature, 1997, 386, 410-414).

PLA and PLGA micro- and nanoparticles have been extensively studied for the encapsulation and release of a large number of therapeutic molecules (Quintanar-Guerrero et al., Drug Dev. Ind. Pharm., 1998, 24 (12), 1113-1128, Sánchez et el., Int. J. Pharm., 1993, 99, 263-273, Sturesson et al., J. Control. Rel., 1999, 59, 377-389, Hsu et al., J. Drug Targ., 7 (4), 313-323). An outstanding characteristic of these particles lies in the fact that their capacity to control the release of active molecules depends on their degradation profile. In this way, a control in the polymer degradation rate has a direct impact on the control of the release of the active molecule associated thereto. It is known that polyester degradation leads to the formation of acid oligomers which may accumulate inside the particles thus causing acidification of the polymeric lattice and, with this, an important reduction in the particles' internal pH (Belbella et el., Int. J. Pharm., 1996, 129, 95-102). This acid microclimate caused by the accumulation of the polymer's degradation products within the particles has a very negative effect on the stability of the active molecule incorporated therein and it represents a limitation in the use of these polymeric systems for the controlled release of macromolecules such as proteins and DNA plasmids (Zhu et al., Nature Biotech., 18, 52-57).

Poloxamers are polyoxyethylene-polyoxypropylene-polyoxyethylene-type triblock copolymers (PEO-PPO-PEO) which, depending on their PEO:PPO ratio vary in their characteristics of molecular weight, hydrophobicity, etc. Poloxamines are copolymers formed from 4 chains of PEO-PPO bound by an ethylen diamine bridge. As with the poloxamers, their characteristics may vary when the PEO-PPO ratio is changed.

One of the applications that has been recently proposed for this family of polyoxyethylene-derived copolymers is to promote the transport of drugs through the blood-brain harrier (BBB) (Kabanov et al., Adv. Drug. Deliv. Rev., 2003, 55, 151-164). Likewise, recent studies have revealed the interest thereof in DNA plasmid transfection studies (Lemieux et al., Gene Ther., 2000, 7, 986-991).

Furthermore, PEO-PPO block copolymers have been widely studied as coating agents which permit modifying the biodistribution of nanoparticles used as drug transporters. Thus, there are numerous works which have revealed that coating nanoparticles with poloxamers and poloxamines affects their biodistribution and, therefore, their capacity to transport drugs to different regions of the organism (Moghimi et al., FEBS Letters, 1994, 344, 25-30, Hawley et al., FEBS Letters, 1997, 400, 319-323).

There are various documents wherein the use of PEO-PPO derivatives are claimed as nanoparticle coating agents (WO96/20698 and US4904479). The object has been to prolong the circulation time thereof after their intravenous injection and to modify their biodistribution profile. In said compositions, the poloxamer/poloxamine does not form part of the polymeric matrix constituting the particles but it is absorbed on a superficial level. Therefore, the quantity of poloxamer/poloxamine absorbed is limited and its presence does not have implications for the encapsulation or controlled release of the active molecule encapsulated in the particles, instead its role is limited to the modification of the particle biodistribution profile.

Additionally, in another document, US5578325, the idea of chemically bonding said copolymers to polyesters, thus forming multi-block copolymers, has been proposed. In these cases, the polyoxyethylenated derivative is covalently bound to the polyester, thus leading to the formation of a new copolymer. These copolymers also permit obtaining PEO-PPO coated nanoparticles which mean they stay in the circulatory stream for a long time after their intravenous administration.

Another of the applications which PEO-PPO block copolymers have been object has been the stabilisation of the proteins encapsulated in the PLGA particles and the modification of their release from same. Thus, in studies previously performed in our laboratory, we have been able to check that the incorporation of PEO-PPO block copolymers, more specifically poloxamers, in micro and nanoparticles of poly-lactic acid/glycolic acid (PLGA) allows the stability of the proteins nanoencapsulated in said particles to be improved. In this initial study, for the incorporation of poloxamer in the particles, we have chosen the double-emulsion method (water/organic solvent/water), according to which, the hydrophilic poloxamer is dissolved in the aqueous internal phase of the emulsion (Blanco et al., Eur. J. Pharm. Biopharm., 1997, 43, 287-294, Blanco et al. Eur. J. Pharm. Biopharm. 1998, 45, 285-294). This method permits the incorporation of very small quantities of poloxamer compared to the quantities of PLGA (normally the ratio is 10:1 PLGA:poloxamer). This is due to two main reasons: on the one hand, the volume of the internal aqueous phase wherein the poloxamer is dissolved is much less than the volume of organic solvent wherein the hydrophobic polymer is dissolved (PLGA); on the other hand, the poloxamer tends to diffuse, during the emulsification process, from the aqueous internal phase towards the aqueous external phase, thus hindering particle formation. This difficulty has been resolved by using an anhydrous microencapsulation method, consisting of the formation of an organic solvent emulsion (wherein the PLGA and the poloxamer have to be dissolved) in an external oily phase wherein a surfactant agent is dissolved. Thanks to this method, high quantities of poloxamers have been incorporated in PLGA microparticles (up to 50%) forming mixed PLGA:poloxamer matrices. This mixed microparticulate system, formed by an intimate mixture of poloxamer and PLGA, has permitted the controlled release of proteins (Tobio et al., Pharm. Res. 1999, 16, 5, 682-688). Nevertheless, the most notable drawback of this method lies in the difficulty in obtaining nanometric particles, the medium size of those particle populations being higher than 1 µm (1000 nanometres). Furthermore, given the necessity of using oils as external phase of the emulsion, it becomes very laborious to isolate the microspheres and large quantities of organic solvents need to be used to eliminate the oil. Therefore, no method has been disclosed to date that allows high quantities of poloxamers to be incorporated in mixed poloxamer:PLGA nanoparticles.

The first reference found relating to the use of poloxamers in the formation of mixed matrices with polyesters based on the physical bonding of both polymers disclosed in the publication US5330768. Said document proposes the use of said mixtures in order to attain a modification in the release of the active molecule incorporated in these systems. Herein, reference is made to the formation of films by co-dissolution of both polymers in a common organic solvent and subsequent evaporation of the solvent or by the joint fusion of both polymers and also to the formation of microparticles by the double-emulsion method (water/organic solvenL/water); nevertheless, the formation of nanoparticles is not mentioned. We should highlight that said method of particle formation in external aqueous phase only allows the incorporation of limited quantities of hydrophilic poloxamers due to their logical tendency to diffuse to the external aqueous phase; we have been able to verify this fact in our previous studies (Blanco et al., Eur. J. Pharm. Biopharm, 1997, 43, 287-294, Blanco et al., Eur. J. Pharm. Biopharm., 1998, 45, 285-294). Likewise, document US5330768 does not mention the use of lipophilic poloxamers or poloxamines in the formation of said mixtures.

The first document found which makes reference to the formation of a mixed microparticulate system formed by an intimate mixture of poloxamer and PLGA designed to improve the stability of microencapsulated proteins, further allowing their controlled release, is the document published by Tobio et al. (Pharm. Res. 1999, 16, 5, 682-688). More recently, document US6465425 also discloses the formation of biodegradable microparticles containing poloxamer with the same purpose. Likewise, said composition incorporates, with the same purpose, an acid-type excipient and at least one polysaccharide. According to said document, the quantity of poloxamer that can be included in said composition can vary between 1-40% with respect to the total weight of the composition. The presentation format of this composition is a film, obtained by simple evaporation of the solvent, or microparticles obtained by atomising. Nevertheless, no reference has been made to the formation of nanoparticles, which can be understood if we bear in mind that the atomising technique does not allow particles as small as nanoparticles to be obtained.

In the same line, with the aim of improving protein stability, we should highlight the document filed by Schwendeman et al., (US2002/0009493), which discloses the use of hydrophilic poloxamers with molecular weight between 500 and 30,000 Da, as pore-forming agents in systems prepared from polyesters. Said document claims the presentation of these compositions in the form of cylinders or microparticles, with a size between 10-100 µm. These particles are obtained using the double-emulsion technique in external aqueous phase, which only permits the incorporation of small quantities of hydrophilic poloxamers, as is indicated in previous studies (Blanco et al., Eur. J. Pharm. Biopharm, 1997, 43, 287-294; Blanco et al., Eur. J. Pharm. Biopharm., 1998, 45, 285-294), or alternatively, using the organic solvent/oil emulsification technique, which, as previously indicated (Tobio et al., Pharm. Res. 1999, 16, 5, 682-688), does not allow nanoparticles to be obtained.

With regard to documents which make explicit reference to the formation of nanoparticles containing poloxamers, we should quote document US5962566. Nevertheless, this document indicates the incorporation of cholesterol as an essential ingredient for nanoparticle formation. The formation method further indicates the need to melt the array of materials and their subsequent dispersion in an aqueous phase.

Likewise, we can cite a document which discloses the formation of nanoparticles which incorporate poloxamers and poloxamines in their structure, in addition to a stabilising lipidic agent (US20030059465). These nanoparticles are intended for the release of the camptothecin cytostatic agent and they are obtained by a process of hydrating previously lyophilised lipids. Although said document claims the possible incorporation of polyesters such as PLCA, the technique disclosed is not applicable to this type of polymers. In any case, the incorporation of lipids in the structure is shown as an essential element of the nanoparticulate composition.

As a consequence of reviewing the previous documents, we should highlight that, despite the large number of documents which make reference to the formation of PLGA and poloxamer particles, none of said documents disclose the formation of mixed matrices which contain high quantities of poloxamers and poloxamines, with different hydrophilic/lipophilic characteristics and which are presented in nanoparticulate form. This last aspect is of critical importance as the microencapsulation techniques designed for the formation of microparticles generally differ from the nanotechnologies applied to the formation of nanoparticles. We should also highlight that the published documents relaLed to obtaining nanoparticles only use hydrophilic poloxamers incorporated in very low proportions in the nanoparticulate system.

### DESCRIPTION OF THE INVENTION

The present invention relates to nanoparticles which comprise a biodegradable polymer, preferably a polyester and a polyoxyethylene-derived block copolymer, preferably poloxamer and poloxamine. The invention further relates Lo a preparation method which permits the incorporation of high percentages of poloxamers and poloxamines in nanoparticles, the biodegradable polymer: polyethylenated derivate ratio being between 1:0.1 and 1:3.

Therefore, according Lo a first aspect, the invention relates to a method of preparing nanoparticles, having a size of less than 1 µm, for the administration of active ingredients, comprising the steps of:
a) dissolving a biodegradable polymer together with a polyoxyethylene-derived block copolymer in an organic solvent, the biodegradable polymer:block copolymer weight ratio being between 1:0.1 and 1:3;
b) adding, with stirring, the solution obtained to a polar phase, wherein the biodegradable polymer has low solubility, precipitating the polymers and forming the nanoparticles;
c) eliminating the organic solvent; d) isolating the particles. The active ingredient can be directly dissolved in the non-polar organic solvent (lipophilic molecules) or it can be previously dissolved in a small volume of aqueous phase (water-soluble molecules) and then dispersed in the organic solvent, before or after stage a).

Preferably, the organic solvent in a) will be a non-polar solvent.

According to a preferred embodiment, the preparation of nanoparticle formulations of intimate mixture may additionally include a lyophilisation stage. In lyophilised form, nanoparticles can be stored for long periods of time and can be easily regenerated by simply adding an optimum volume of water. The nanoparticle lyophilisation has been optimised by incorporating a cryoprotective excipient (glucose or trehalose) in the formulations' suspension medium.

According to another form of preferred embodiment, in the previous method, the biodegradable polymer is a polyester, which is selected from the group of polyesters such as polylactic acid, polylactic co-glycolic acid and their copolymers, polycaprolactone or the group of polyanhydrides. The polylactic co-glycolic acid polymer 50:50 Resomer® RG 503 Mw: 35000 (Boehringer lngelheim) has been used to prepare the nanoparticles of intimate mixture.

According to other forms of preferred embodiment, the block copolymer is selected from poloxamers and poloxamines.

Poloxamers are polyoxyethylene-polyoxypropylene-polyoxyethylene-type triblock copolymers (PEO-PPO-PEO) which, depending on their PEO:FPO ratio, vary in their characteristics of molecular weight, hydrophobicity, etc. Preferably, the poloxamers used will have a molecular weight between 1,000 and 25,000 Daltons. These polymers can be obtained from BASF Corporation with the trade name Pluronic.TM. For the preparation of nanoparticles of intimate mixture, we have used the following poloxamers: Pluronic.TM F68 with molecular weight 8350 and HLB=29, Pluronic.TM with molecular weight 4400 and HLB=1.

Poloxamines are copolymers formed from 4 chains of PEO-PPO bound by an ethyl diamine bridge. As with the poloxamers, their characteristics may vary when the PEO-PPO ratio is changed. Preferably, the poloxamines used will have a molecular weight between 1,000 and 25,000 Daltons. These polymers can be obtained from BASF Corporation with the trade name Tetronic.TM. For the preparation of nanoparticles of intimate mixture, we have used the following poloxamines: Tetronic.TM 908, with molecular weight 25000 and HLB=30.5, Tetronic.TM 904, with molecular weight 6700 and HLB=14.5 and Tetronic.TM 901, with molecular weight 4700 and HLB=2.5

According to another preferred embodiment, the proportion by weight of the biodegradable polymer is between 1:1 and 1:3.

According to a second aspect of the present invention, it relates to both lyophilised and non-lyophilised nanoparticles obtained according to the aforementioned method.

These nanoparticles offer innovative, distinctive characteristics given their capacity for the encapsulation and controlled release of very delicate active molecules, such as proteins and DNA plasmids. Furthermore, due to the presence of important amounts of poloxamers and poloxamines in their composition, said nanoparticles can present a differentiated biodistribution profile, in comparison with the classic particles formed from polyesters.

Due to their nanoparticulate size, these new systems can be administered to the human organism by any administration route, including intravenous route, whilst microparticles cannot be administered by this route due to the obstruction they would cause in the blood capillaries. There is also a great quantity of documentation which shows that nanoparticles can overcome biological barriers (mucous membranes, epithelials) whilst microparticles cannot.

The physicochemical properties of the formulations of different composition and different polymer ratio have been characterised using Photon Correlation Spectroscopy (PCS) and Laser Doppler Anemometry techniques. The nanoparticle morphology was studied using transmission electronic microscopy (TEM) and 1H NMR. These studies confirmed the formation of the aforementioned intimate mixture system.

In order to check the applicability of these nanoparticles of intimate mixture for the release of derivate macromolecules, we have encapsulated the pEGFP-C1 plasmid (coder for a green fluorescent protein) in the different formulations. The results of these *in vitro* release studies have revealed the potential of the formulations as controlled release vehicles during extended periods of time.

The cytotoxicity of nanoparticles of different compositions, at different concentrations, has been tested in cell cultures with the MTS colorimetric test ((3-(4,5-dimethylthiazol-2-yl)-5-(3-carboxymethoxyphenyl)-2-(4-sulphophenyl)-2H-tetruzolium) in the MCF-7 cell line grown in Dulbecco's Modified Eagle's Medium (DMEM) supplemented with 1.0% foetal bovine serum (FBS). It can be concluded that none of the formulations produce toxic effects in the cells.

According to a third aspect, the present invention relates to compositions, especially pharmaceutical and cosmetic, which incorporate nanoparticles according to the present invention.

Below, the invention will be explained in greater detail, based on a series of examples, without limitative character for the scope of the invention.

### EXAMPLES

### DESCRIPTION OF THE FIGURES

- FIG. 1:: 1H NMR spectra of the PLGA/Pluronic.TM F68 nanoparticles formulations with different polymer ratios.
- FTG. 2:: TEM images of the PLGA/Pluronic.TM F68 nanoparticle formulations with polymer 1:1 ratio.
- FIG. 3:: 1H NMR spectra of the formulations of the PLCA/Tetrollic.TM 908 nanoparticles with different polymer ratios.
- FIG. 4:: TEM images of the PLGA/ Tetronic.TM 908 nanoparticle formulations with 1:1 polymer ratio.

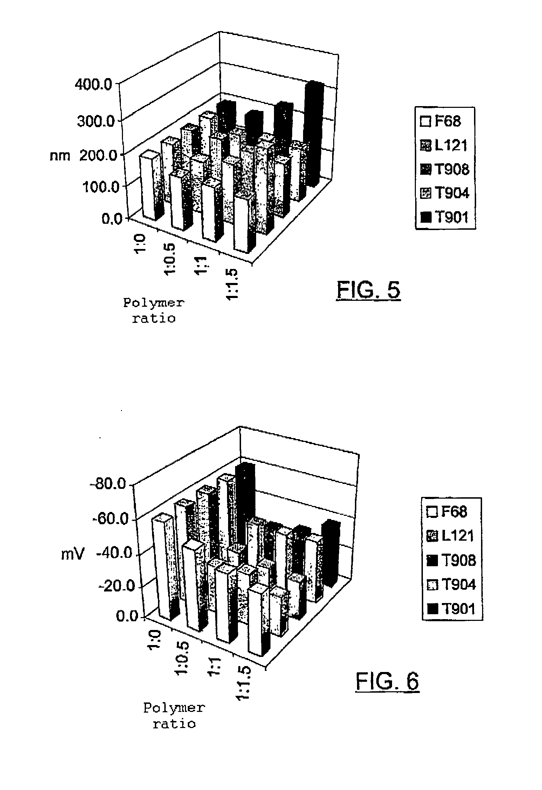

- FIG. 5:: PLGA/poloxamer and PLGA/poloxamine nanoparticle size in accordance with the PLGA/polymer ratio and the type of poloxamer or poloxamine.
- FIG. 6:: surface charge of the PLGA/poloxamer and PLGA/poloxamine nanoparticles in accordance with the PLGA/polymer ratio and the type of poloxamer or poloxamine.
- FIG. 7:: effect of the cryoprotective agents on lyophilised PLGA/poloxamer nanoparticle size.
- FIG. 8:: effect of the cryoprotective agents on lyophilised PLGA/poloxamer nanoparticle size.
- FIG. 9:: *in vitro* release profile of plasmidic DNA encapsulated in the PLGA/F68, PLGA/L121, PLGA/T908 and PLGA/T904 nanoparticles with 1:1 polymer ratio.

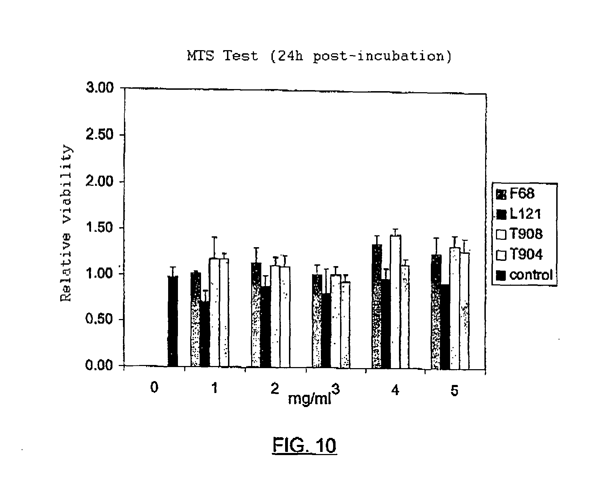

- FIG. 10:: results of the cytoxicity test of the PLGA/F68, PLGA/L121, PLGA/T908 and PLGA/T904 nanoparticles with 1:1 polymer ratio in the MCF-7 cell culture.

### EXAMPLE 1

Nanoparticles of intimate mixture were prepared with the aforementioned modified solvent diffusion technique. More specifically: 50 mg of polylactic co-glycolic acid and 25, 50 or 75 mg of Pluronic.TM F68 (HLB=29) poloxamer were dissolved in 2 ml of dichloromethane and this organic solution was mixed for 30 sec. by vortex (2400 min⁻¹, Heidolph), with a small volume of aqueous phase. The emulsion thus obtained was added to 25 ml of ethanol with moderate magnetic stirring. The formulation was diluted with 25 ml of water and it was stirred for a further 10 minutes. After solvent evaporation at 30°C and in a vacuum (Rotavapor, Büchi R-114), the nanoparticles were collected and were concentrated in aqueous medium. Optionally, for their later analysis, the nanoparticles were centrifuged (1 h, 8000xg, 15°C, Avanti 30, Beckman) and they were lyophilised (48 hours at -34°C, Labconco Corp).

The size and polydispersion of the nanoparticles were measured using photon correlation spectroscopy (PCS) and the surface charge was determined by Laser Doppler Anemometry (Zetasizer 3000 HS, Malvern Instruments) (TABLE 1).

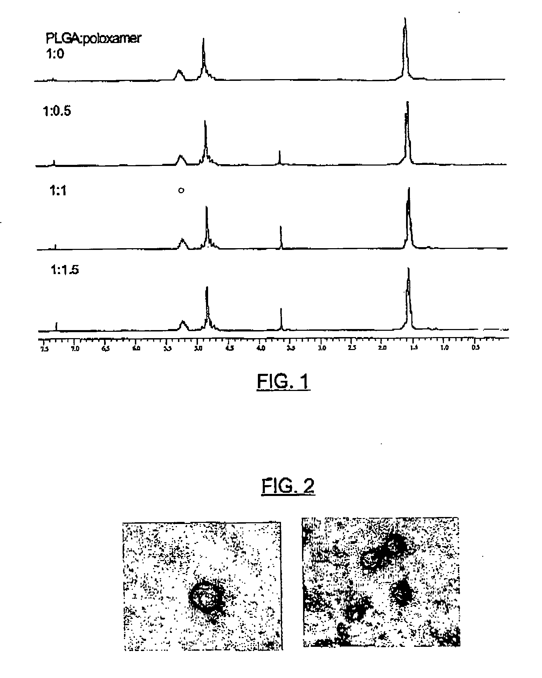

The matrix composition was analysed using 1H NMR spectroscopy (Bruker AMX-300) from lyophilised samples dissolved in deuterated chloroform. These studies confirmed the presence of the poloxamer/poloxamine in the nanoparticulate matrix. From the intensities of the corresponding peaks, we can also conclude that the quantity of polyoxyethylene-polyoxypropylene block copolymers can be changed by adjusting the preparation parameters. (FIGURE 1).

The morphological analysis of the nanostructures was performed using transmission electronic microscopy (CM 12 Philips) using samples stained with a 2% phosphotungstic acid solution. (FTGURE 2).

**TABLE 1**

| | Pluronic.TM F68 | | |
|---|---|---|---|
| PLGA:poloxamer | Size (nm) | P.I | ζ-pot. (mV) |
| 1 : 0 | 191.5±7.1 | 0.046 | -60.117.4 |
| 1 : 0.5 | 162.8±4.4 | 0.079 | -50.2±0.8 |
| 1 : 1 | 163.2±5.1 | 0.135 | -43.1+6.4 |
| 1 : 1.5 | 159.8±6.5 | 1.163 | -38.5±0.6 |

### EXAMPLE 2

Nanoparticles of intimate mixture were prepared with the aforementioned modified solvent diffusion technique, but changing the type of polyoxyethylene-polyoxypropylene copolymer. The PLGA and the different quantities of Pluronic.TM L121 (HLB-1) poloxamer were dissolved in dichloromethane and this organic solution was mixed by vortex with a small volume of aqueous phase. The emulsion thus obtained was added to ethanol with stirring. The formulations were diluted with water and it was stirred for a further 10 minutes. After the solvent evaporation, the nanoparticles were concentrated in aqueous medium. Optionally, for their later analysis, the nanoparticles were centrifuged and lyophilised. The size and polydispersion of the nanoparticles were measured using PCS and the surface charge was determined by Laser Doppler Anemometry (TABLE 2). The morphology and composition of the matrices were studied using 1H NMR spectroscopy and TEM microscopy.

**TABLE 2**

| | Pluronic.TM L121 | | |
|---|---|---|---|
| PLGA:poloxamer | Size (nm) | P.I | ζ-pot. (mV) |
| 1 : 0 | 191.5±7.1 | 0.046 | -60.1±7.4 |
| 1 : 0.5 | 164.5±6.3 | 0.156 | -27.3±7.1 |
| 1 : 1 | 185.5±6.0 | 0.195 | -30.0±8.0 |
| 1 : 1.5 | 257.3±10.0 | 0.179 | -24.5±5.5 |

### EXAMPLE 3

Nanoparticles of intimate mixture were prepared with the aforementioned modified solvent diffusion technique, but changing the type of polyoxyethylene- polyoxypropylene copolymer: the PLGA and the different quantities of Tetronic.TM 908 (HLB=30.5) poloxamine were dissolved in dichloromethane and this organic solution was mixed by vortex with a small volume of aqueous phase. The emulsion thus obtained was added to ethanol with stirring. The formulations were diluted with water and it was stirred for a further 10 minutes. After the solvent evaporation, the nanoparticles were concentrated in aqueous medium.

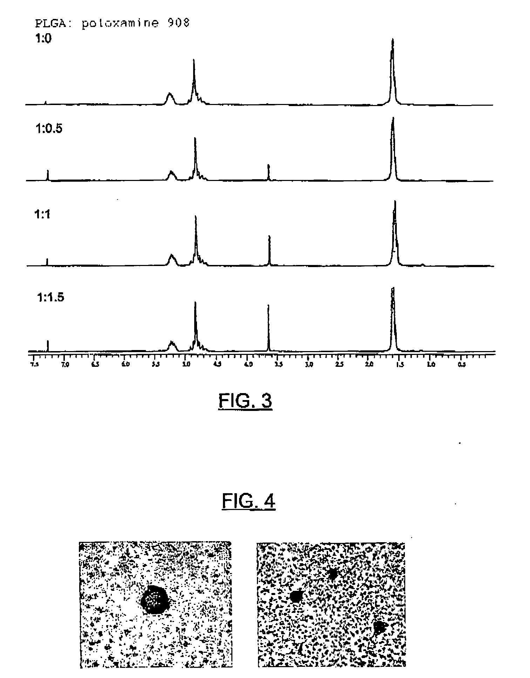

Optionally, for their later analysis, the nanoparticles were centrifuged and lyophilised. The size and polydispersion of the nanoparticles were measured using PCS and the surface charge was determined by Laser Doppler Anomometry (TABLE 3). The morphology and composition of the matrices were studied using 1H NMR spectroscopy and TEM microscopy (FIGURE 3 and 4)

**TABLE 3**

| | Tetronic.TM 908 | | |
|---|---|---|---|
| PLGA:poloxamine | Size (nm) | P.I | ζ-pot. (mV) |
| 1 : 0 | 191.5±7.1 | 0.046 | -60.1±7.4 |
| 1 : 0.5 | 189.2±4.6 | 0.202 | -30.9±3.9 |
| 1 : 1 | 174.0±5.4 | 0.271 | -26.9±1.2 |
| 1 : 1.5 | 171.2±3.2 | 0.235 | -24.1±1.0 |

### EXAMPLE 4

Nanoparticles of intimate mixture were prepared with the aforementioned modified solvent diffusion technique, but changing the type of polyoxyethylene- polyoxypropylene copolymer: the PLGA and the different quantities of Tetronic.TM 904 (HLB-14.5) poloxamine were dissolved in dichloromethane and this organic solution was mixed by vortex with a small volume of aqueous phase. The emulsion thus obtained was added to ethanol with stirring. The formulations were diluted with water and it was stirred for a further 10 minutes. After the solvent evaporation, the nanoparticles were concentrated in aqueous medium.

Optionally, for their later analysis, the nanoparticles were centrifuged and lyophilised. The size and polydispersion of the nanoparticles were measured using PCS and the surface charge was determined by Laser Doppler Anemometry (TABLE 4). The morphology and composition of the matrices were studied using 1H NMR spectroscopy and TEM microscopy (FIGURES 3 and 4)

**TABLE 4**

| | Tetronic.TM 904 | | |
|---|---|---|---|
| PLGA:poloxamine | Size (nm) | P.I | ζ-pot. (mV) |
| 1 : 0 | 191.5±7.1 | 0.046 | -60.1±7.4 |
| 1 : 0.5 | 160.2±5.6 | 0.188 | -40.0±4.6 |
| 1 : 1 | 168.7±9.4 | 0.179 | -38.4±3.3 |
| 1 : 1.5 | 168.8±2.5 | 0.160 | -39.6±2.0 |

### EXAMPLE 5

Nanoparticles of intimate mixture were prepared with the aforementioned modified solvent diffusion technique, but changing the type of polyoxyethylene- polyoxypropylene copolymer: the PLGA and the different quantities of Tetronic.TM 904 (HLB=14.5) poloxamine were dissolved in dichloromethane and this organic solution was mixed by vortex with a small volume of aqueous phase. The emulsion thus obtained was added to ethanol with stirring. The formulations were diluted with water and it was stirred for a further 10 minutes. After the solvent evaporation, the nanoparticles were concentrated in aqueous medium.

Optionally, for their later analysis, the nanoparticles were centrifuged and lyophilised. The size and polydispersion of the nanoparticles were measured using PCS and the surface charge was determined by Laser Doppler Anemometry (TABLE 5). The morphology and composition of the matrices were studied using 1H NMR spectroscopy and TEM microscopy.

**TABLE b**

| | Tetronic.TM 901 | | |
|---|---|---|---|
| PLGA:poloxamine | Size (nm) | P.I | ζ-pot. (mV) |
| 1 : 0 | 191.5±7.1 | 0.046 | -60.1±7.4 |
| 1 : 0.5 | 205.3±54.5 | 0.162 | -25.4±5.0 |
| 1 : 1 | 277.4±102.9 | 0.308 | -28.9±5.4 |
| 1 : 1.5 | 333.7±82.1 | 0.275 | -38.2±8.3 |

### EXAMPLE 6

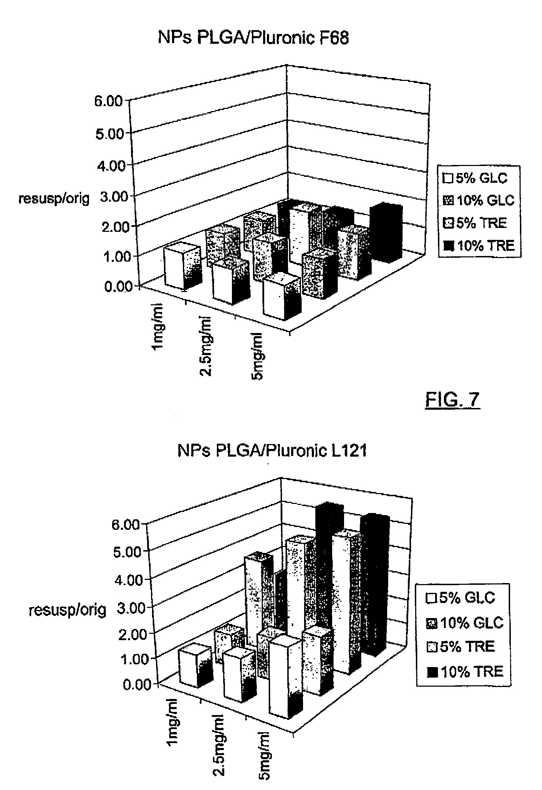

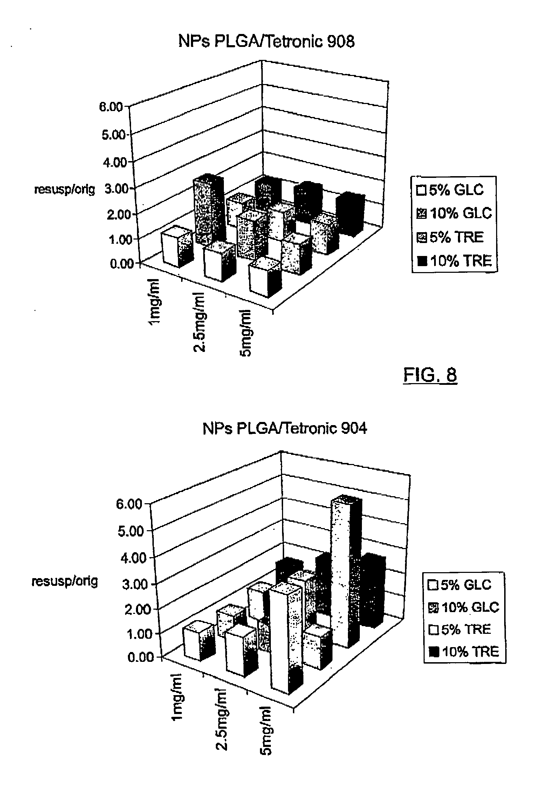

Nanoparticles of intimate mixture of PLGA/Pluronic.TM F68, PLGA/Pluronic.TM L121, PLGA/Tetronic.TM 908 and PLGA/Tetronic.TM 904 with 1:1 polymer ratio were prepared as has been described in examples 1, 2, 3 and 4. Two cryoprotective agents (glucose and trehalose) were incorporated in the nanoparticulate suspension medium. The formulations, at different concentrations (1, 2.5, 5 mg/ml), were lyophilised in the presence of 5% or 10% of the cryoprotective agent. The nanoparticle size and polydispersion were measured after the lyophilisation/resuspension process and they were compared with the initial values. The effects of the nanoparticle concentration, the type and the concentration of the cryoprotective agent have been evaluated. Tt can be concluded that in the presence of 5% cryoprotective agent, all the formulations can be lyophilised at relatively high concentrations (2.5 mg/ml) without significant aggregation (FIGURE 7 and 8) .

**TABLE 6**

| Cryoprotectiv e agent | Dilution of NPs Mg/ml | Resuspended /original size ratio | | | |
|---|---|---|---|---|---|
| | | F68 | L121 | T908 | T904 |
| 5% glucose | 1 | 1.2110.04 | 1.25+0.21 | 1.17±0.04 | 1.18±0.01 |
| | 2.5 | 1.15±0.06 | 1.65±0.29 | 1.12±0.09 | 1.59±0.35 |
| | 5 | 1.11±0.09 | 2.62±0.73 | 1.03±0.05 | 3.77±0.39 |
| 10% glucose | 1 | 1.28±0.04 | 1.32±0.39 | 2.60 | 1.04 |
| | 2.5 | 1.39±0.15 | 1.69±0.67 | 1.59 | 1.18 |
| | 5 | 1.30±0.10 | 2.25±0.15 | 1.20 | 1.33 |
| 5% trehalose | 1 | 1.22±0.17 | 3.58±2.47 | 1.13±0.04 | 1.29±0.17 |
| | 2.5 | 1.93±0.55 | 4.66±2.9 | 1.21±0.03 | 2.27±0.90 |
| | 5 | 1.57±0.27 | 5.23±0.21 | 1.23±0.04 | 5.66±4.48 |
| 10% trehalose | 1 | 1.21±0.12 | 2.32±1.09 | 1.25±0.10 | 1.64 |
| | 2.5 | 1.30±0.07 | 5.46±0.99 | 1.46±0.06 | 2.33 |
| | 5 | 1.82±0.51 | 5.35±10.52 | 1.46±0.20 | 2.74 |

### EXAMPLE 7

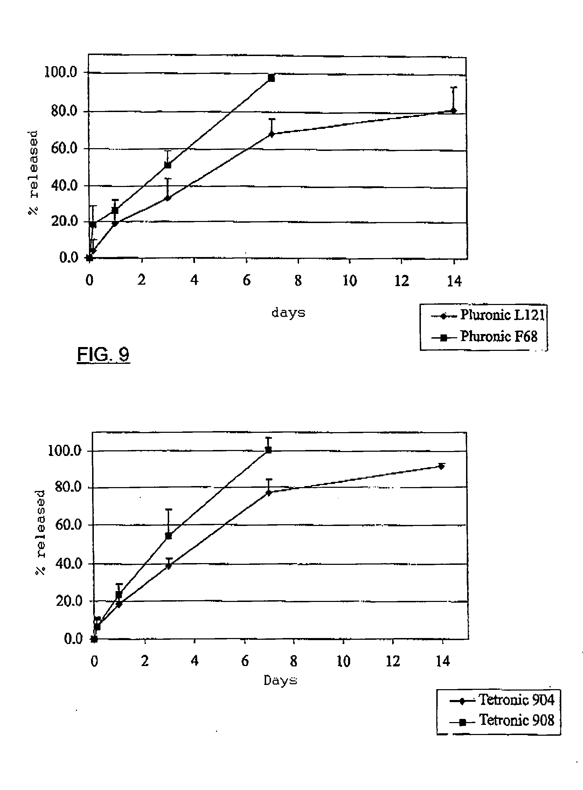

Nanoparticles of intimate mixture of PLGA/Pluronic.TM F68, PLGA/Pluronic.TM L121, PLGA/Tetronic.TM 908 and PLGA/Tetronic.TM 904 with 1:1 polymer ratio were prepared as has been described in examples 1, 2, 3 and 4. The pEGFP-C1 plasmid model (coder of a green fluorescent protein) was incorporated in the internal aqueous phase of the formulations with a theoretical charge of 0.4%. The size, polydispersion and surface charge of the formulations loaded with DNA were measured using photon correlation spectroscopy and Laser Doppler Anemometry (TABLE 7). The encapsulation efficiency and the *in vitro* release profiles were determined from the samples of supernatants of different times with fluorometric assays using the PicoGreen Quantification Kit (Molecular Probes) in TE buffer at pH = 7.5 (FIGURE 9).

**TABLE 7**

| Type of poloxamer/poloxamine | Size (nm) | P.I | ζ- potential (mV) | Encapsulation efficiency (%) |
|---|---|---|---|---|
| Pluronic.TM F68 | 182.6±6.0 | 0.114 | -50.8±3.6 | 35.2±8.9 |
| Pluronic.TM L121 | 216.8±5.3 | 0.154 | -23.5±1.4 | 31.3±3.8 |
| Tetronic.TM T908 | 268.7±11.6 | 0.437 | -35.0±0.9 | 32.0±3.7 |
| Tetronic.TM T904 | 161.5±7.6 | 0.154 | -54.1±2.0 | 44.1±4.3 |

### EXAMPLE 8

Nanoparticles of intimate mixture of PLGA/Pluronic.TM F68, PLGA/Pluronic.TM L121, PLGA/Tetronic.TM 908 and PLGA/Tetronic.TM 904 with 1:1 polymer ratio were prepared as has been described in examples 1, 2, 3 and 4. The cytotoxicity of the formulations was studied in the MCF-7 cell culture in DMEM supplemented with 10% FBS. The cells were incubated with different nanoparticle concentrations (from 1 to 5 mg/ml) for 24 hours. The cell viability was measured with MTS reagent after a 24-hour recovery period. The results show that, despite the high concentrations and extended incubation times, none of the formulations produce toxic effects in the cells.

## Claims

1. Method of preparing nanoparticles, having a size of less than 1 µm, for the administration of active ingredients, **characterised in that** it comprises the steps of:
a) dissolving a biodegradable polymer together with a polyoxyethylene-derived block copolymer in an organic solvent, the weight ratio of both polymers being between 1:0.1 and 1:3;
b) adding, with stirring, the solution obtained to a polar phase, wherein the biodegradable polymer has low solubility, precipitating the polymer and forming the nanoparticles;
c) eliminating the organic solvent;
d) isolating the particles
where the active ingredient is dissolved in the organic solvent used in a) before or after step a), or is dissolved in a small volume of the aqueous phase, which is then dispersed in the organic solvent used in a), before or after step a).

2. Method according to claim 1, **characterised in that** it comprises an additional step after e) of lyophilising the nanoparticles obtained.

3. Method according to any of claims 1 and 2, **characterised in that** the biodegradable polymer is a polyester.

4. Method according to any of claims 1 and 2, **characterised in that** the biodegradable polymer is a polyanhydride.

5. Method according to claim 3, **characterised in that** the polyester is selected from polycaprolactone, polylactic acid, polylactic co-glycolic acid and their mixtures.

6. Method according to any of claims 1 to 5, **characterised in that** the block copolymer is a poloxamer.

7. Method according to claim 6, **characterised in that** the poloxamer has a molecular weight comprised between 1,000 and 25,000 Daltons.

8. Method according to any of claims 1 to 5, **characterised in that** the block copolymer is a poloxamine.

9. Method according to claim 8, **characterised in that** the poloxamine has a molecular weight comprised between 1,000 and 25,000 Daltons.

10. Method according to any of claims 1 to 9, **characterised in that** the active ingredient is selected from molecules with therapeutic properties, vaccinations and cosmetic ingredients.

11. Method according Lo any of claims 1 to 10,
**characterised in that** the weight ratio of both polymers is between 1:1 and 1:3.

12. Nanoparticles for the administration of pharmaceutically- or cosmetically-active ingredients, having a size of less than 1 µm, which can be obtained using the method according to any of claims 1 and 3 to 10.

13. Lyophilised nanoparticles for the administration of pharmaceutically- or cosmetically-active ingredients, having a size of less than 1 µm, which can be obtained using the method according Lo claim 2.

14. Compositions **characterised in that** they comprise nanoparticles, according to any of claims 12 and 13.

15. Pharmaceutical or cosmetic compositions, **characterised in that** they comprise nanoparticles, according to any of claims 12 and 13.
